# FASCICULE DE BREVET EUROPEEN

(11) **EP 1 879 602 B1**
(45) Date de publication et mention de la délivrance du brevet: **01.06.2016**
(21) Numéro de dépôt: 06755465.9
(22) Date de dépôt: 28.04.2006
(51) Int. Cl.: A61K 36/185, A61K 8/97

(54) **UTILISATION DE POLYPHENOLS DE CACAO POUR REGULER LA PIGMENTATION DE LA PEAU**
VERWENDUNG VON KAKAOPOLYPHENOLEN ZUR STEUERUNG DER HAUTPIGMENTIERUNG
USE OF CACAO POLYPHENOLS FOR CONTROLLING SKIN PIGMENTATION

(30) Priorité: 03.05.2005 FR 0504493
(43) Date de publication de la demande: 23.01.2008
(73) Titulaire: SOCIETE DE RECHERCHE COSMETIQUE SARL, 2314 Luxembourg (LU)
(72) Inventeur: LECLERE, Jacques, F-45500 Saint-Gondon (FR)
(74) Mandataire: L'Helgoualch, Jean
(86) Numéro de dépôt international: PCT/FR2006/000965
(87) Numéro de publication internationale: WO 2006/117465

(56) Documents cités:
- WO-A-2004/080380
- FR-A- 2 654 935
- FR-A- 2 810 242
- FR-A- 2 851 916
- US-A1- 2003 175 234
- US-A1- 2004 096 566
- US-A1- 2004 180 102
- SHIMOGAKI H ET AL: "In vitro and in vivo evaluation of ellagic acid on melanogenesis inhibition" INTERNATIONAL JOURNAL OF COSMETIC SCIENCE, KLUWER ACADEMIC PUBLISHERS, DORDRECHT, NL, vol. 22, 2000, pages 291-303, XP009021427 ISSN: 0142-5463

## Description

La présente invention concerne une nouvelle utilisation de polyphénols extraits de cacao en cosmétique et en dermatologie, et plus particulièrement une nouvelle utilisation de polyphénols extraits de cacao pour assurer la régulation de la pigmentation de la peau.

La peau constitue un véritable organe comprenant plusieurs couches intégrées, allant de la couche superficielle, l'épiderme, jusqu'aux couches plus profondes, le derme et l'hypoderme, et chacune possède des propriétés spécifiques permettant à l'ensemble de réagir et de s'adapter aux conditions de son environnement.

L'épiderme, principalement composé de kératinocytes (90% des cellules épidermiques), de mélanocytes (2 à 3% des cellules épidermiques) et des cellules de Langerhans, a une épaisseur variable selon les différentes parties du corps.

Le derme, plus épais, se compose principalement de collagène, d'élastine et de protéoglycanes. Ces trois types de molécules sont synthétisés par les fibroblastes dermiques. Les fibres de collagène assurent la résistance mécanique et la texture de la peau, l'élastine est responsable de l'élasticité, et les protéoglycanes jouent un rôle majeur de structure et d'hydratation de la peau. D'autres cellules comme les macrophages et les leucocytes sont également présentes dans la couche du derme.

L'hypoderme, qui est la couche la plus profonde de la peau, contient les adipocytes qui produisent des lipides pour que le tissu sous-cutané fabrique une couche grasse protégeant les muscles, les os et les organes internes contre les chocs.

La pigmentation de la peau résulte de la présence de mélanine dans l'épiderme et le derme. La mélanine est produite par les mélanocytes situés principalement dans la couche basale, en présence de la tyrosinase (ou monophénol mono-oxygénase), une enzyme cupro-protéique qui catalyse la transformation de la tyrosine en dihydroxyphénylalanine (dopa) qui est ensuite transformée en dopaquinone, puis en dopachrome, pour aboutir à la mélanine suivant un mécanisme complexe. Cette biosynthèse, ou mélanogenèse, est un processus complexe qui est aujourd'hui relativement bien connu.

Dans des conditions normales, la pigmentation de la peau est uniforme. Toutefois, on observe fréquemment l'apparition d'une hyperpigmentation, c'est-à-dire une pigmentation excessive localement, qui peut se manifester par des taches de rousseur, lentigo sénile, mélasme, des taches de vieillissement, une pigmentation due à une exposition excessive au soleil, une hyperpigmentation post-inflammatoire due à l'abrasion, des dermatites, etc. A l'inverse, il est parfois souhaitable de favoriser la pigmentation de la peau pour des raisons esthétiques ou pour lutter contre les effets de certaines affections, comme par exemple le vitiligo, qui se traduisent par un défaut localisé de pigmentation.

L'hyperpigmentation de la peau peut être combattue au moyen d'un composé susceptible de dégrader la mélanine, ou d'inhiber la biosynthèse de la mélanine, ou encore en utilisant un inhibiteur de tyrosinase qui bloque ou inhibe le mécanisme décrit ci-dessus. Ainsi, l'hydroquinone et certains de ses dérivés peuvent agir en inhibant la tyrosinase et en empêchant la fixation de son substrat naturel, la tyrosine. Ainsi, le brevet EP-A-060.092 décrit l'utilisation d'un ester d'hydroquinone et d'acide gras, et le brevet US-A-4.692.261 décrit une composition dépigmentante contenant de l'hydroquinone, un détergent anionique et un stabilisant. On connaît également des associations à base d'hydroquinone, comme par exemple l'association d'hydroquinone et d'acide salicylique décrite dans le brevet FR-A-2.754.253. Cependant, l'hydroquinone présente l'inconvénient d'une forte toxicité entraînant des effets secondaires tels qu'une irritation de la peau.

D'autres substances dépigmentantes ont été proposées, et par exemple des dérivés de mélatonine comme dans le brevet FR 2.751.535, des dérivés d'acide salicylique comme dans le brevet FR 2.732.594, des extraits de plantes du genre Mitra-carpus comme dans le brevet FR 2.751.874, ou encore des esters d'acide kojique présentant une action éclaircissante sur la peau, comme décrit dans le brevet FR 2.460.131. Des extraits de Glycyrrhiza contenant des substances telles que la glabridine, ont aussi été utilisés pour inhiber la production de mélanine par les cellules du derme en bloquant la tyrosinase mise en oeuvre dans sa synthèse, et des compositions dépigmentantes contenant de la glabridine ont été proposées comme dans le brevet FR 2.822.067.

On sait aussi que de nombreuses substances favorisent la mélanogénèse et la pigmentation de la peau, et par exemple des caroténoïdes et certaines vitamines telles que la vitamine E et la vitamine C. On connaît aussi, comme composés favorisant la synthèse de la mélanine, des alpha-hydroxy acides comme dans la demande WO 99.51198, des extraits de plantes, notamment *Sanguisorba officinalis* comme dans le brevet EP 993.826, ou encore des composés activant l'expression de la tyrosinase comme des psoralènes.

La demande WO 03082227 décrit une composition destinée à réguler la mélanogénèse, qui comprend à la fois des promoteurs et des inhibiteurs de la synthèse de la mélanine.

Certains polyphénols ont aussi été proposés pour renforcer la pigmentation de la peau. Ainsi, la demande de brevet FR 2.851.916 décrit l'utilisation de polyphénols catéchiques afin de favoriser la pigmentation naturelle de la peau, et par exemple des extraits de cacao utilisables dans des compositions pour administration par voie orale. Des extraits de santhines, éventuellement incorporés dans des liposomes, peuvent être utilisés dans des compositions cosmétiques et dermatologiques favorisant la pigmentation de la peau, et le brevet FR 2.654.935 décrit des extraits de xanthines obtenues à partir de graines de cacaoyer.

Par ailleurs, le brevet EP 1.289.491 enseigne que les polyphénols extraits du cacao (Theobroma cacao) possèdent des propriétés intéressantes utilisables dans des compositions cosmétiques et dermatologiques, notamment une activité anti-ride.

On distingue trois groupes de polyphénols de cacao, qui appartiennent tous à la famille des flavonoïdes : les caté-chines (flavanols, 37% env.) et notamment la (-)épicatéchine, les procyanidines (oligomères flavanoliques, 58%) et les anthocyanes (5%).

Les études réalisées par la demanderesse ont montré que les polyphénols extraits du cacao pouvaient aussi avoir une influence sur la tyrosinase et la mélanine, et exercer une activité de régulation de la pigmentation de la peau qui peut se manifester soit en inhibant la pigmentation, soit en la favorisant, selon la dose utilisée.

De plus, on a constaté que, de manière inattendue, l'effet sur la pigmentation de la peau pouvait s'inverser en fonction de la dose utilisée. Ainsi, à faible dose, l'effet est inhibiteur de la pigmentation, tandis qu'à dose élevée il est promoteur de la pigmentation. Ces résultats permettent alors de proposer des compositions à base de polyphénols de cacao capables de jouer un rôle régulateur de la pigmentation de la peau, et plus particulièrement d'inhiber la pigmentation de la peau.

La présente invention décrit une nouvelle utilisation de polyphénols extraits de cacao pour la préparation d'une composition cosmétique destinée à réguler la pigmentation de la peau.

L'invention décrit une nouvelle utilisation de polyphénols extraits du cacao pour la préparation d'une composition permettant d'inhiber la pigmentation de la peau.

L'invention décrit une nouvelle composition topique spécialement adaptée à une telle utilisation, comprenant un extrait de polyphénols de cacao ou des cellules de cacao en une concentration adaptée à l'effet recherché.

Les études effectuées ont montré l'intérêt des polyphénols de cacao, tant comme extraits de cacao (Theobroma cacao), éventuellement encapsulés, que comme cellules de cacao.

Ces études ont notamment montré, comme indiqué plus loin, que les polyphénols de cacao suivant l'invention
- présentent une parfaite innocuité vis-à-vis des cellules endothéliales de l'épiderme, les images histologiques des épidermes traités étant similaires à celles des témoins non traités ;
- entraînent une diminution significative du taux de la mélanine et de l'activité de la tyrosinase au niveau des explants de peau humaine traités, à la concentration de 0,5% ;
- entraînent une augmentation du taux de la mélanine et de l'activité de la tyrosinase, à la concentration de 2%.

Ainsi, les compositions topiques décrites dans l'invention peuvent être utilisées avantageusement en cosmétique pour assurer la régulation de la pigmentation de la peau chez des sujets susceptibles d'être affectés par un dérèglement pouvant se manifester par une hyperpigmentation.

Les compositions décrites dans la présente invention peuvent se présenter sous toutes les formes galéniques usuelles adaptées à une application topique, et de préférence sous forme de lotion, crème, lait ou sérum.

Elles peuvent comprendre moins de 1% en poids d'extrait de polyphénols de cacao, par rapport au poids total de la composition, lorsqu'un effet dépigmentant est recherché. A l'inverse, une concentration de plus de 1% en poids, par exemple entre 1,5 et 5% en poids, est utile lorsqu'un effet pigmentant est recherché. Dans le cas de l'utilisation de cellules de cacao, les compositions peuvent comprendre moins de 0,10% en poids de cellules de cacao, par rapport au poids total de la composition, lorsqu'un effet dépigmentant. Une concentration de plus de 0,10% en poids, par exemple entre 0,15 et 0,5% en poids, procure un effet pigmentant.

Comme indiqué ci-dessus, au lieu d'utiliser des extraits de polyphénols de cacao, on peut utiliser des cellules de cacao obtenues par des techniques connues de croissance ou multiplication cellulaire, en milieu liquide ou solide, de cellules d'origine végétale cultivées *in vitro* dans un environnement contrôlé, dans des conditions aseptiques en l'absence de micro-organismes. Ainsi, on pourra se référer aux travaux effectués sur des suspensions cellulaires de cacao tels que ceux de Gurney et Evans, Exp. Bot. 43, 769-775, (1992), Wen et Kinsella, J. Food Sc. 57, 1452-1457 (1992) et Leathers et Scragg, Plant Sc. 62, 217-228 (1989). Ainsi, on peut plus particulièrement utiliser des cellules dédifférenciées lyophilisées de cacao dont les teneurs des trois principaux composants sont respectivement de 1,2 mg d'anthocyanes, 136,0 mg d'oligomères procyanidoliques (OPC) et 11,2 mg d'épicatéchine, par gramme de cellules lyophilisées. La lyophilisation des cellules permet de faciliter leur conservation et, par suite, leur incorporation dans les compositions cosmétiques.

Les compositions décrites dans la présente invention peuvent être présentées sous les formes classiquement utilisées pour une application topique, c'est-à-dire sous forme de lotion, gel, émulsion (en particulier crème ou lait), masque, pommade, liposomes ou encore des patches transdermiques, contenant des excipients et supports usuels compatibles et pharmaceutiquement acceptables. Elles peuvent aussi se présenter sous forme de lingettes imbibées d'une solution contenant un extrait de polyphénols de cacao suivant l'invention.

Ces formes d'administration par voie topique sont préparées par les techniques usuelles, et par exemple, dans le cas d'une crème, par dispersion d'une phase grasse dans une phase aqueuse pour obtenir une émulsion huile dans eau, ou inversement pour préparer une émulsion eau dans huile. Dans le cas de crèmes, on préfère utiliser des émulsions à structure lamellaire contenant peu de produits éthoxylés ou n'en contenant pas du tout.

Les compositions topiques peuvent par exemple comprendre des excipients appropriés pour une administration topique externe, en particulier des excipients acceptables sur le plan dermatologique et cosmétologique. Ces excipients appropriés pour la formulation sont bien connus de l'homme du métier et comprennent en particulier des agents de pénétration tels que l'éthoxydiglycol, le phytantriol, l'octyl dodécanol et l'escine ; les épaississants tels que les gommes naturelles et les polymères de synthèse ; les émollients et les tensioactifs tels que l'octanoate de cétéaryle, le myristate d'isopropyle, l'isononanoate de cétéaryle, la diméthicone, la cyclométhicone, le 3-diisostéarate de polyglycéryle, le polyisobutène hydrogéné, l'alcool cétylique, le palmitate cétylique, le phosphate cétylique ; les émulsionnants tels qu'une lécithine ou un tensioactif à base d'inuline comme l'Inutec^{®} de la société Orafti ; les conservateurs tels que le phénoxyéthanol, le parahydroxybenzoate de méthyle (méthylparaben), le parahydroxybenzoate d'éthyle (éthylparaben), le parahydroxybenzoate de propyle (propylparaben) et le Phenonip® associant du phénoxyéthanol et des parahydroxybenzoates de méthyle, éthyle, butyle et isobutyle ; les colorants ; les parfums ; etc.

D'autres ingrédients peuvent être utilisés dans les compositions : les agents hydratants tels que le propylène glycol, la glycérine, le butylène glycol et également les vitamines antioxydantes telles que la vitamine E, par exemple l'acétate de tocophérol ou le tocotriénol, la vitamine C, les polyphénols naturels. On peut également ajouter à la composition des agents conditionneurs de la peau tels que le nylon et le nitrure de bore.

Des agents de protection contre les rayons ultraviolets peuvent aussi être avantageusement incorporés dans les compositions, et par exemple des filtres solaires UV-A et UV-B hydrophiles ou lipophiles, choisis parmi la benzophénone ou un dérivé de benzophénone tel que la 2-hydroxy-4-méthoxy-benzophénone (Eusolex® 4360), ou un ester d'acide cinnamique et plus particulièrement le méthoxycinnamate d'octyle (Eusolex® 2292), le méthoxycinnamate d'éthyl-2-hexyle (Parsol MCX®), ou encore un cyano-β,β-diphénylacrylate tel que l'octocrylène (Eusolex® OCR), le 4-méthylbenzylidène camphre (Eusolex 6300®), et des dérivés du dibenzoylméthane tels que le 4-isopropyl dibenzoylméthane (Eusolex 8020), le t-butylméthoxy dibenzoylméthane (Parsol 1789®), et le 4-méthoxydibenzoylméthane. On peut aussi utiliser des pigments formant écran anti-ultraviolet, comme par exemple le dioxyde de titane, l'oxyde de zinc, l'oxyde de zirconium ou encore l'oxyde d'aluminium.

Des exemples non limitatifs de compositions conformes à la présente invention sont donnés ci-après. Sauf indication contraire, les pourcentages et parties sont indiqués en poids.

### Exemple 1

Suivant les techniques classiques, on prépare un gel dépigmentant régulateur de la pigmentation ayant la composition pondérale suivante.

| | |
|---|---|
| EDTA trisodique | 0,10 |
| Pentylène glycol | 5,00 |
| Caprylyl glycol | 3,00 |
| Sorbate de potassium | 0,30 |
| Carbomer | 0,30 |
| Hydroxyde de sodium | 0:10 |
| Extrait polyphénolique de cacao | 0,50 |
| Extrait de thé vert | 1,00 |
| Eau de roses | 20,00 |
| Eau déminéralisée | qsp 100,00 |

L'extrait polyphénolique utilisé dans la composition ci-dessus est un extrait obtenu par broyage de fèves de cacao et séparation hydrophile/lipophile du beurre de cacao d'une part et d'un mélange de protéines et de polyphénols d'autre part. La séparation hydrophile / lipophile permet, par extraction de la graine broyée, de séparer à l'eau les corps gras surnageants de la phase polyphénols / protéines. On utilise de préférence une eau portée à une température comprise entre 80 et 100°C environ.

Le gel ayant la composition indiquée ci-dessus est utilisé en application sur les bras et le visage, une à deux fois par jour pendant une période de 4 à 6 semaines.

### Exemple 2

Une crème de régulation de la pigmentation de la peau ayant la composition pondérale suivante peut être préparée de manière usuelle.

| | |
|---|---|
| Eau déminéralisée | qsp 100,00 |
| EDTA trisodique | 0,10 |
| Pentylène glycol | 5,00 |
| Octyl dodécanol | 3,00 |
| Polyacrylate de sodium | 1,00 |
| Caprylique / capriques triglycérides | 4,00 |
| Palmitate de cétyle | 2,00 |
| Alcool béhénylique | 3,00 |
| Polysorbate 60 | 3,50 |
| Stéarate de sorbitan | 2,50 |
| Extraits de polyphénols de cacao | 2,00 |
| Guggulipides | 0,50 |
| Essence de géranium | 0,02 |
| Essence d'orange amère | 0,10 |
| Essence d'aiguilles de pin | 0,03 |
| Conservateur | 0,15 |

### Exemple 3

### Evaluation de la cytotoxicité

La cytotoxicité de l'extrait de polyphénols vis-à-vis des cellules endothéliales a été évaluée comme suit.

L'extrait polyphénolique de l'invention, aux concentrations de 0,5% et 2% en poids, et un placebo (même base sans l'extrait de polyphénol) sont déposés à raison de 10 µl/cm² sur des explants de peau humaine pendant 24 heures, comparativement à des explants témoins. Cet essai sur quatre lots (deux suivant l'invention, un témoin et un placebo) a été conduit en duplicate.

On constate que la composition de l'invention n'induit aucune toxicité.

Les images histologiques, après coloration HES des explants traités sont similaires à celles des explants témoins.

Ces résultats montrent que l'extrait de polyphénols ne présente aucune activité cytotoxique vis-à-vis des cellules endothéliales de l'épiderme, quelle que soit la concentration.

### Exemple 4

### Evaluation de l'effet sur la pigmentation

L'activité sur la pigmentation (effet dépigmentant et effet pigmentant) a été étudiée à travers
- l'évaluation de l'activité de la tyrosinase
- l'évaluation de la synthèse de la mélanine (étude qualitative de la mélanine intracellulaire par spectrométrie à 475 nm)

L'étude a été faite sur des épidermes reconstitués (Skinetic^{®}). Des kératinocytes d'origine humaine sont cultivés dans un milieu défini (MCDB 153 modifié) et supplémentés. Les cellules sont cultivées pendant 10 jours à l'interface air/liquide, le milieu de culture étant changé tous les deux jours.

Les épidermes ainsi formés sont mis en co-culture avec des mélanocytes de phototypes II, IV et VI sur des filtres en polycarbonate. L'étude est réalisée entre le 11^{ème} et le 17^{ème} jour de la culture.

Six lots ont été constitués :
- Lot 1 : épiderme témoin (témoin négatif) ne recevant aucun produit.
- Lot 2 : épiderme témoin (témoin positif) traité par un dépigmentant connu (acide kojique)
- Lot 3 : épiderme traité par la composition à base d'extrait de polyphénols de l'invention, à 0,5%.
- Lot 4 : épiderme traité par la composition à base d'extrait de polyphénols de l'invention, à 2%.
- Lot 5 : épiderme traité par des cellules de cacao (0,05%).
- Lot 6 : épiderme traité par des cellules de cacao (0,10%).

L'essai est conduit en triplicate après 3 jours de contact.

### Dosage de la mélanine :

L'évaluation de la synthèse de la mélanine intracellulaire (étude qualitative) a été effectuée comme indiqué ci-dessus par spectrométrie à 475 nm après digestion des explants de peau humaine puis dissolution dans NaOH 1N et du diméthyl sulfoxyde pendant 30 minutes.

Les résultats sont regroupés dans le tableau ci-après.

| | Absorbance (475 nm) | % |
|---|---|---|
| Témoin négatif | 0,185 ± 0,04 | - |
| Témoin positif (acide kojique) | 0,107 ± 0,01 | -42 |
| Extrait de polyphénols de cacao 0,5% | 0,145 ± 0,03 | -21 |
| Extrait de polyphénols de cacao 2% | 0,215 ± 0,02 | +16 |
| Cellules de cacao 0,05% | 0,134 ± 0,07 | -27 |
| Cellules de cacao 0,10% | 0,223 ± 0,09 | +20 |

Ces résultats montrent que la composition à base d'extrait de polyphénols à la dose de 0,5%, entraîne une diminution significative du taux de mélanine, tandis que, au contraire, elle entraîne une augmentation significative du taux de mélanine à la concentration plus élevée de 2%. Il en est de même pour les cellules de cacao, aux concentrations de 0,05% et 0,10% respectivement.

### Evaluation de l'activité tyrosinasique :

A la fin de la période d'incubation, le milieu de culture a été prélevé, puis les épidermes ont été rincés avec du PRS et mis en contact du Triton X-100 (Sigma, France) à 1% puis incubés pendant 10 minutes. La réaction enzymatique a été initiée par l'addition de L-dopamine (Sigma, France) à 10 mM dans du PBS dépourvu de Ca²⁺ et de Mg²⁺.

Après 1 heure d'incubation à 37°C à l'abri de la lumière, l'activité de la tyrosinase a été évaluée par la mesure de l'absorption à 475 nm au moyen d'un spectrophotomètre.

Les résultats sont regroupés dans le tableau ci-après.

| | Absorbance (475 nm) | % |
|---|---|---|
| Témoin négatif | 0,235 ± 0,05 | - |
| Témoin positif (acide kojique) | 0,122 ± 0,03 | -48 |
| Extrait de polyphénols de cacao 0,5% | 0,186 ± 0,03 | -20 |
| Extrait de polyphénols de cacao 2% | 0,275 ± 0,09 | +17 |
| Cellules de cacao 0,05% | 0,175 ± 0,07 | -25 |
| Cellules de cacao 0,10% | 0,274 ± 0,04 | +16 |

Ces résultats confirment que la composition sous forme d'extrait de polyphénols ou de cellules de cacao, entraîne une diminution significative du taux d'activité de la tyrosinase aux faibles concentrations (0,5% et 0,05% respectivement) tandis qu'elle entraîne une augmentation de l'activité de la tyrosinase aux concentrations plus élevées (2% et 0,10% respectivement).

Ainsi, la composition présente une parfaite innocuité vis-à-vis de l'épiderme et possède la particularité de présenter une activité dépigmentante ou au contraire pigmentante, selon la concentration.

## Revendications

1. Utilisation non-thérapeutique d'une composition cosmétique topique, comprenant un extrait de polyphénols de cacao (Theobroma cacao) à une concentration de 0,5% en poids par rapport au poids total de la composition ou des cellules de cacao à une concentration de 0,05% en poids par rapport au poids total de la composition, pour la dépigmentation de la peau.

2. Composition cosmétique à usage topique à effet dépigmentant, **caractérisée en ce qu'**elle comprend un extrait de polyphénols de cacao à une concentration de 0,5 % en poids ou des cellules de cacao à une concentration de 0,05% en poids, par rapport au poids total de la composition.

## Patentansprüche

1. Nichttherapeutische Verwendung einer topischen kosmetischen Zusammensetzung, umfassend einen Kakaopolyphenolextrakt (Theobroma cacao) mit einer Konzentration von 0,5 Gew.-% im Verhältnis zum Gesamtgewicht der Zusammensetzung oder Kakaozellen mit einer Konzentration von 0,05 Gew.-% im Verhältnis zum Gesamtgewicht der Zusammensetzung für die Depigmentierung der Haut.

2. Kosmetische Zusammensetzung zur topischen Anwendung mit depigmentierender Wirkung, **dadurch gekennzeichnet, dass** sie einen Kakaopolyphenolextrakt (Theobroma cacao) mit einer Konzentration von 0,5 Gew.-% im Verhältnis zum Gesamtgewicht der Zusammensetzung oder Kakaozellen mit einer Konzentration von 0,05 Gew.-% im Verhältnis zum Gesamtgewicht der Zusammensetzung umfasst.

## Claims

1. The non-therapeutic use of a topical cosmetic composition, comprising a cocoa polyphenol extract (*Theobroma cacao)* at a concentration of 0.5% by weight based on the total weight of the composition or cocoa cells at a concentration of 0.05% by weight based on the total weight of the composition, for depigmentation of skin.

2. A cosmetic composition for topical use with a depigmenting effect, **characterized in that** it comprises an extract of cocoa polyphenols at a concentration of 0.5% by weight or of cocoa cells at a concentration of 0.05%, based on the total weight of the composition.
